# EUROPEAN PATENT SPECIFICATION

(11) **EP 2 066 255 B1**
(45) Date of publication and mention of the grant of the patent: **23.06.2010**
(21) Application number: 06821710.8
(22) Date of filing: 25.09.2006
(51) Int. Cl.: A61C 1/05, A61C 1/07, A61B 17/00, A61B 17/16, A61B 17/32, A61C 3/03, A61C 8/00, A61B 17/34

(54) **HANDPIECE WITH SURGICAL TOOL TO PERFORM HOLES IN BONE TISSUES**
HANDSTÜCK MIT OPERATIONSWERKZEUG ZUR ANBRINGUNG VON LÖCHERN IN KNOCHENGEWEBEN
EMBOUT À MAIN AVEC OUTIL CHIRURGICAL POUR RÉALISER DES TROUS DANS DES TISSUS OSSEUX

(43) Date of publication of application: 10.06.2009
(73) Proprietor: PIEZOSURGERY S.R.L., 16039 Sestri Levante (GE) (IT)
(72) Inventor: VERCELLOTTI, Domenico, 16039 Sestri Levante (GE) (IT); VERCELLOTTI, Tomaso, 16033 Lavagna (GE) (IT); BIANCHETTI, Fernando, 16043 Chiavari (GE) (IT)
(74) Representative: Petruzziello, Aldo
(86) International application number: PCT/IT2006/000680
(87) International publication number: WO 2008/038307

(56) References cited:
- EP-A- 1 380 265
- EP-A1- 0 456 470
- EP-A1- 0 482 195
- WO-A-2005/056224
- WO-A1-99/34121
- DE-A1-102005 009 802
- DE-U1- 20 115 678
- GB-A- 2 029 289
- US-A- 4 823 793
- US-A- 5 514 141
- US-A- 5 674 235
- US-A1- 2002 099 400
- US-A1- 2002 107 519
- US-A1- 2002 156 492
- US-A1- 2006 004 396
- US-A1- 2007 015 102
- US-B2- 6 695 847

## Description

The present invention refers to a handpiece with a surgical tool to perform holes of various shapes in bone tissues, adapted to receive various fixing systems (single screws, screws for fixing plates) and/or dental implants.

According to the prior art, the sites (that is the holes or the seats) for the insertion of screws and/or of various fixing systems in the bone are prepared through the use of rotating instruments or tools driven by micromotors. Said rotating tools generally have helical shaped tips (twist-drills), cutters or reamers.

Document WO 2005 056 224 A discloses an ultrasound handpiece according to the preamble of claim 1.

These instruments, however, have severe limitations, especially when they are used in complex anatomical situations, in particular:
- when there is a limited surgical access, which makes the correct preparation of the hole in the bone difficult;
- in the presence of anatomically delicate bone structures; and
- in proximity to soft tissues (nerves, blood vessels), with the consequent risk of injury.

Another limiting aspect of such rotating instruments is represented by the high mechanical energy produced by the rotation, which requires a certain pressure to be applied to the tool, causing friction loss and thus heat generation. This results in a risk of overheating of the tissues involved in the operation, with possible impairment of healing.

Normally, the amount of heat generated by friction is directly related to the intensity of the pressure applied to the rotating tool, to the speed of rotation, to the size and to the shape of the tip/cutter/reamer and to the time taken to make the hole. Perforation of the bone therefore involves the use of irrigation to reduce the heat generated. This irrigation can be external or internal.

Moreover, the irrigating solution must be able to act on the whole contact surface present in the bone-tool interface, that is, on the cutting front part and on the side part. This action might not be achieved if the tool (the cutter) is not removed from the hole every so often, so as to allow both the removal of the bone fragments and the entry of the irrigating fluid into the site.

It must be considered that the temperature increase is not caused only by the incomplete accessibility of the cooling fluid into the hole, but also by the obstruction caused by bone debris depositing on the cutting edges of the cutter, which makes the drilling action less efficient and prolongs the time necessary to make the hole. Furthermore, the rotating action of the cutter compresses the bone debris against the wall of the hole, forming a smear layer, which obstructs the natural sinuses of the spongy bone to the detriment of osteoregenerative processes.

Cutters rotating at low speeds (1500 - 1800 rpm) require the operator to apply a considerable pressure on the handpiece (from 1.8 to 2.5 kg) in order to cut the bone. This gives rise to two types of problem:

### a) Reduced surgical control.

The operator must exert a considerable pressure during the drilling action, which is not compatible with the precision, especially when passing through a bone tissue with uneven mineralization. This aspect leads to a considerable risk of injury in delicate anatomical situations, such as near vessel or nerve endings which, in contact with the cutting action of the rotating cutters/tips, may be torn.

### b) Overheating.

The macro vibrations of the rotating tools give rise to overheating of the bone surface, which spreads centrifugally into the bone part surrounding the hole. This fact, together with the presence of bone debris, which remains in the sinuses, slows and/or limits the bone regeneration.

Object of the present invention is to overcome the drawbacks of the prior art by providing a handpiece with a special surgical tool, so that the particular geometry of the tool and its mode of operation allow holes to be made in the bone tissue with extreme precision.

Another object of the present invention is to provide such a handpiece with a surgical tool for making holes in a bone tissue that is versatile and able to form holes of shapes other than circular, according to requirements.

Another object of the present invention is to provide such a handpiece with a surgical tool for making holes in bone tissue that is able to improve the subsequent osteoregenerative processes.

These objects are achieved in accordance with the invention with the characteristics listed in appended independent claim 1.

Advantageous embodiments of the invention are apparent from the dependent claims.

According to the invention, the handpiece for performing holes in bone tissues comprises a surgical tool provided with a tip (or head) adapted to make a hole in the bone. The handpiece works by ultrasound, and the tip of the tool comprises a plurality of cutting elements defining the profile of the hole to be made in the bone. A main channel which ends in an outlet hole opening into the tool tip is provided in the body of the tool for the passage of a cooling fluid, so as to cool the working area affected by the tool tip.

The ultrasound handpiece provided with the tool according to the invention presents the following advantages with respect to the prior art:

### 1. Greater precision.

The action of the conventional devices (micromotors combined with tips, cutters) is associated with macro vibrations, which make the performance of the operation imprecise, whilst that of the ultrasound handpiece according to the invention is characterised by micro vibrations of the tools, which allow the operator a greater tactile sensitivity and a greater intraoperative precision. The handpiece according to the invention, thanks to the ultrasonic micro vibrations of the tool and to the special design of the cutting elements and of the outlets between the cutting elements of the tip of the tool, produces holes in the bone through a process of micronization of the tissue, which is removed immediately by the mechanical action of the irrigation fluid which, subjected to ultrasonic vibrations, produces a cavitation effect, the result of which is a perfect cleansing of the bone surface representing the seat of the hole. The removal and irrigation action is also supported by the particular geometrical shape of the tip. Removal of the bone takes place through micro vibrations. In this manner the centrifugal overheating effect is less extensive than that produced by the macro vibrations generated by rotation of the tips/cutters.

### 2. Greater stability of the tool at the start of the drilling

Rotating tools are unstable at the start of the drilling because of a centrifugal drift component, which causes the tool to deviate from the desired drilling axis. In fact, according to the prior art, in the field of implant surgery in order to engage the bone surface to be drilled a special tip is used (commonly known as a rose tip) to produce an entry guide hole. Instead, the particular configuration of the tool tip according to the invention makes it possible to give greater stability. In fact the tool tip has a concave type sharpening with cusp-shaped cutting elements protruding peripherally towards the tip. Thanks to this configuration of the tool tip and to its ultrasonic vibrations, at the time of starting the hole in the bone there is no centrifugal drift component that causes the tool to deviate from the desired drilling axis.

### 3. Greater cleaning of the tool/bone interface and consequent improvement in osteoregenerative processes.

According to the invention, the particular geometry of the tool tip (longitudinal outlets in the side surface of the tip) together with the ultrasonic vibrations which cause the cavitation effect of the irrigation fluid, allow the removal of the bone debris from the side walls of the hole made by the tool, leaving the tool/bone interface clean. In this manner the typical smear layer of the twist drills and of the cutters is not formed, thus favouring osteoregenerative processes.

### 4. Smaller temperature increase, due to the friction between the tool and the bone on the work surfaces during the drilling of the bone.

The tool according to the invention has an axial duct, which allows the passage of the irrigation liquid, which flows out through a central hole in the tool tip and washes away the bone debris, carrying it through the radial channels of the tip until it reaches the longitudinal outlets in the tip, which allow the removal of the debris. In this manner a considerable temperature reduction is achieved in the work area. Furthermore, the cleansing and cooling action performed by the tip on the sidewalls of hole is enhanced by the presence of a second lateral duct situated near the tip, which allows the outflow of the irrigation liquid. Furthermore, the ultrasonic frequency micro vibrations of the tool, which cause the cavitation phenomenon of the irrigation fluid, contribute to the washing of the walls of the hole formed by the tool.

### 5. Selective drilling of the bone tissues.

Ultrasonic micro vibrations at low frequency (from 20 KHz to 30 KHz) act on the tool according to the invention, which are therefore optimal for drilling the bone tissue but ineffective for soft tissues, contact with which does not cause any tearing action but only a momentary release of heat. These vibrations are not able to cut mineralised tissues. In fact it is known that ultrasonic vibrations capable of cutting soft tissues use a greater frequency (50/60 KHz). Therefore the tools according to the invention, thanks to their particular geometric/structural shape and to the fact that they work at ultrasonic frequencies, are capable of making holes in the bone material through the action of micro vibrations acting on the cutting edges (not through the rotary action typical of the tools used in the prior art) with obvious clinical advantages.

### 6. Reduction of the sources of contamination during the surgical procedure.

Lastly, the ultrasound handpiece with the tools according to the invention, not having rotating parts, reduces the number of possible sources of contamination during the surgical procedure compared with the conventional systems with cutters. In fact in the conventional art the tips/cutters etc. are driven by micromotors, which require lubrication of the transmission members.

Further characteristics of the invention will be made clearer by the detailed description that follows, referring to purely exemplifying and therefore non limiting embodiments thereof, illustrated in the appended drawings, in which:
Figure 1 is a perspective view illustrating an ultrasound handpiece in which a surgical tool according to the invention is mounted;
Figure 2 is a perspective view of the tool of Figure 1;
Figure 3 is an axial sectional view of the tool of Figure 2;
Figure 4 is an enlarged perspective view of the tip of the tool of Figure 2;
Figure 5A is a front view of the tool of Figure 2, in which the tip has been omitted;
Figure 5B is a variance of the shank of Figure 5A;
Figure 6 is a perspective view of a second embodiment of the tool according to the invention;
Figure 7 is an axial sectional view of the tool of Figure 6;
Figure 8 is an enlarged perspective view of the tip of the tool of Figure 6;
Figures 9A and 9B are two diagrammatic views obtained by means of a finished elements (FE) analysis, illustrating the dynamic behaviour of the tool of Figure 2 during a compression and extension cycle, respectively;
Figure 10 is a graph illustrating the oscillating movement of the tool of Figure 2 during a vibration cycle in an x-y plane;
Figures 11A e 11B are two diagrammatic views like Figures 9A and 9B, but illustrating the dynamic behaviour of the insert of Figure 6;
Figures 12A and 12B are two diagrammatic views illustrating the dynamic behaviour of the structure of the tool of Figure 6 obtained respectively by means of a finished elements (FE) analysis and by means of an experimental modal analysis (EMA).

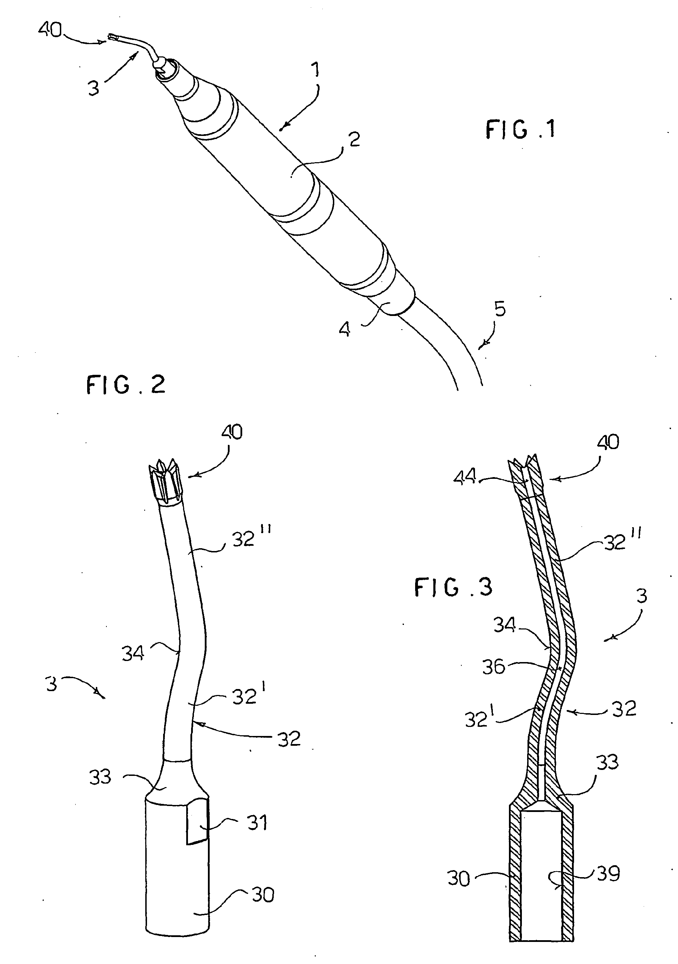

In Figure 1 a surgical device 1, such as an ultrasound handpiece like that illustrated in US patent 6,695,847 cited here as a reference, is illustrated. The handpiece 1 comprises a body 2, substantially cylindrical in shape so that it can be gripped easily by a surgeon. At the top of the body 2 a tool 3 having a shape suitable for drilling a bone and thus for creating an implant site is mounted.

The body 2 of the handpiece is connected to an external connector member 4. The external connector 4 carries electrical and hydraulic supply cables 5 destined to be connected respectively to an electrical power supply, to a hydraulic supply and to a peristaltic pump provided on a console. The console provides a control panel for operation of the handpiece 1.

A transducer connected to the tool 3 is provided inside the handpiece 1. The transducer is preferably of the piezoelectric type and can be a piezoceramic resonator able to convert the electrical input signal into a vibration in the ultrasonic frequency so as to make the tool 3 vibrate. The oscillating frequency goes from 25 kHz to 30 kHz. A basic working ultrasonic frequency of 27 KHz is preferably chosen.

According to the requirements, the supply signal of the transducer having a basic ultrasonic frequency can be modulated or overmodulated with a low frequency signal (6-40 Hz); or it can be modulated or overmodulated with low frequency bursts.

This technique, which uses the modulation of the vibration of the tool 3, allows the heat that develops in the soft tissues because of the dissipation of energy due to vibration of the tool to be minimized.

The method providing for use of a basic signal at ultrasonic frequency modulated with low frequency bursts makes it possible to have a hammering effect of the insert 3, together with an efficacy of the ultrasonic vibration that causes a clean, precise cut in the mineralised tissue, for the formation of a hole in the bone.

With particular reference to Figures 2, 3, and 4, the tool 3 comprises a cylindrical tang 30 to be connected to the ultrasound transducer inside the handpiece 1. The tang 30 comprises two outer grooves 31, parallel to each other and disposed in diametrically opposite positions, adapted to be engaged by a dynamometric key for assembly on the handpiece 1. Said tang 30 has an inside thread 39 to be fixed correctly to the transducer of the handpiece 1.

The tang 30 is connected at the front to a smaller diameter shank 32 by means of a tapered transition element 33 whose diameter decreases going from the tang 30 to the shank 32. The shank 32 has at its distal end a tip or a head 40 composed of a plurality of cutting elements 43. The tip 40 is the working part of the tool 3.

The shank 32 has a cylindrical body whose diameter decreases (from 2.2 mm to 1.8 mm, preferably from 2.00 mm to 1.7 mm) from the transition element 33 towards the tip 40. The shank 32 has a curved intermediate portion 34 which divides it into a first proximal part 32' and a second distal part 32". The main reason for this shape/configuration of the shank 32 is related to optimisation of the vibration in view of the needs of the anatomy of the surgical site.

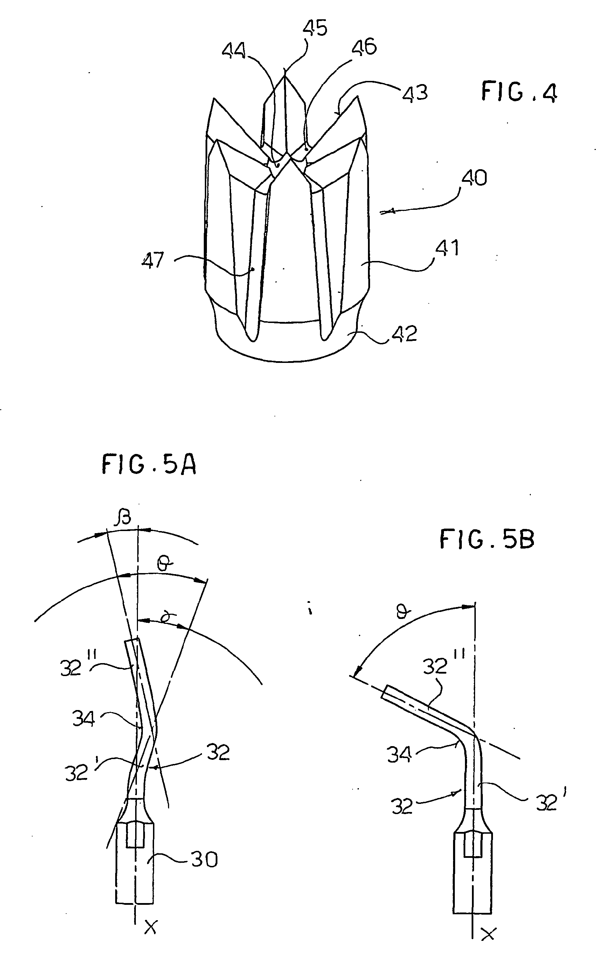

As shown in Figures 5A and 5B, the curved portion 34 of the shank defines an obtuse angle of 180° - 0, which can range from 90° to 180° (excluding the extremes) and is preferably between 110° and 150°. In Figure 5A a shank 32 is illustrated in which the axis of the proximal part 32' of the shank forms an angle α of about 20° with respect to the axis X of the tang 30 and the axis of the distal part 32" of the shank forms an angle β of about 13° with respect to the axis X of the tang. As a result an angle θ of about 33° is defined between the axes of the proximal part 32' and the distal part 32". Therefore the curved part 34 of the shank defines an obtuse angle of 147°.

In a variance illustrated in Figure 5B, the axis of the proximal part 32' of the shank coincides with the axis X of the tang 30 and the axis of the distal part 32" of the shank forms an angle θ of about 64° with respect to the axis X of the tang. Therefore the curved part 34 of the shank defines an obtuse angle of 116°.

As better illustrated in Figure 4, the tip 40 is formed from a cylinder 41, which is connected to the shank by means of a tapered member 42. Cutting elements or teeth 43, specially sharpened and arranged in a substantially circular configuration, are formed on this cylindrical part 41 of the tip.

As shown in Figure 3, a duct 36, which extends for the whole length of the tool and ends in the centre of the tip 40, is formed axially in the body of the tool 3. Said duct 36 is open in the proximal part of the tang 30 and allows the passage of an irrigation fluid, such as, for example, physiological saline coming from the handpiece 1.

The duct 36 ends in an outlet hole 44 at the centre of the tip 40. Therefore the fluid leaving the hole 44 of the tip allows the interface area between the bone tissue and the cutting part of the tip 40 to be irrigated and cooled directly. At the same time, the physical effect of the cavitation (produced by the ultrasonic micro vibrations) is exploited in said interface between the bone tissue and the cutting part of the tip 40, allowing a greater cleaning of the surgical site and a better cooling.

Returning to Figure 4, the cutting elements 43 protrude radially from the outlet hole 44 of the tip. The cutting elements 43 are preferably 6 in number, evenly spaced from each other by an angle of 60°.

The peripheral edges of the cutting elements 43 form cusps 45 protruding towards the distal end of the tool. The cusps 45 of the cutting elements define a circumference having a diameter ranging from 1.8 mm to 2.5 mm, preferably 2.0 mm.

Each cutting element 43 has an irregular pyramid or a wedge shape with a cutting profile inclined with respect to the axis of the tip. Each tooth 43 therefore has a cutting profile which converges radially from the periphery (that is, from the cusp 45 of the tooth) to the central outlet hole 44.

The particular sharpening process used to form the cutting teeth 43 leaves/produces, between adjacent teeth, an outlet which defines a radial channel 46 which starts from the central outflow hole 44 and extends radially towards the outer edge of the tip 40. Therefore, the irrigating liquid which flows axially from the outflow hole 44 (in the centre of the tip) branches out through said radial channels 46 thus allowing the cooling of the cutting area to be maximised and the discharge of the engaged/cut material to be facilitated.

In the outside surface of the cylindrical part 41 of the tip, between one tooth and the other, an outlet is further formed, which defines a longitudinal channel 47 which starts from the radial channel 46 and branches out longitudinally towards the shank. These particular longitudinal channels 47 allow an easy removal of the cut bone material.

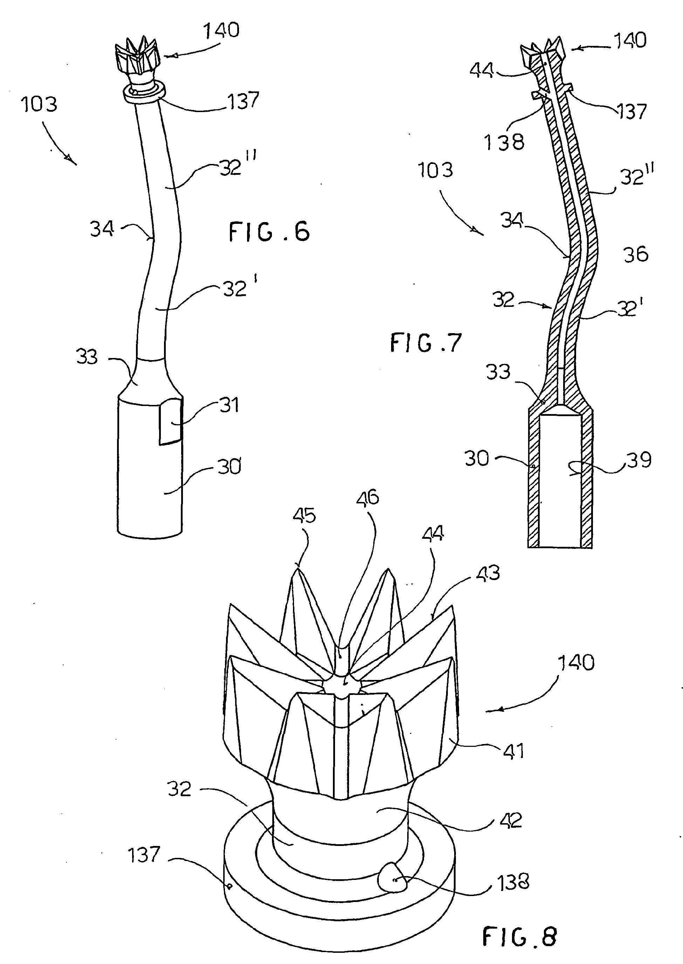

With reference to figures 6 - 8 a tool 103 according to a second embodiment of the invention is described, in which like or corresponding elements to those already described in the first embodiment are designated by the same reference numerals and are not described in detail.

With reference to Figure 8, the tool 103 had a tip 140 slightly different with respect to the tip 40 of the tool 3 of the first embodiment. In fact eight cutting elements or teeth, evenly spaced from each other by an angle of 45°, protrude radially from the outflow hole 44 of the tip 140. In this case, the cusps 45 of the teeth define a circumference having a diameter ranging from 2.8 mm to 4.5 mm, preferably 3.15 mm.

The tool 103 furthermore has a circular ring or collar 137 situated on the shank 32 near the tapered portion 42 of the tip 140. The outside diameter of the ring 137 is substantially equal to or slightly smaller (about 1/10 mm smaller) than the diameter of the circumference defined by the cusps 45 of the cutting elements. The purpose of the ring 137 is to help to keep the direction of drilling of the tool 103 congruent with that performed with the tool 3 of the first embodiment which has a tip 40 with a smaller diameter.

As shown in Figure 7, a lateral irrigation duct 138 communicating with the main irrigation duct 36 and inclined with respect thereto by an angle between 5° and 90°, preferably 45°, is provided level with said ring 137. This lateral duct 138 opens in the side surface of the shank 32 between the ring 137 and the tip 140 and allows the sidewall 41 of the cutting area to be cooled and the bone material removed from said wall to be eliminated.

Even if in the figures the ring 137 and the lateral irrigation duct 138 are illustrated only in the tool 103 of the second embodiment, it is obvious that they can be provided in any type of tool according to the invention.

It should be noted that the handpiece 1 according to the invention has cutting tools (3, 103) vibrating at ultrasonic frequencies to make holes in the bone tissue. Unlike the instruments used for the same purpose in the prior art, the tools 3 and 103 do not have to rotate and therefore, if equipped with a suitable tip, allow holes of various shapes, besides circular, to be made.

It must be considered that endosseous fixing systems (screws/pins etc.) are currently available on the market only for circular holes. In fact the rotating instruments currently available can make holes only with a circular section. For this reason, in the figures, some possible tools (3, 103) with a cylindrical shaped tip (for holes with a circular section) have been illustrated by way of example.

However, other geometries of the tool tips (for holes of shapes other than circular) are possible, using the handpiece 1 according to the invention which exploits ultrasonic micro vibrations and non-rotary movements.

To perfect the shape and the dimensions of the tool according to the invention, a finite element (FE) digital model representing the structure of the ultrasound handpiece coupled to the tool was realised, and then simulations of the dynamic behaviour of said FE model when subjected to ultrasonic vibration were done. To validate the simulations of the dynamic behaviour of the tool made with the FE method, an experimental modal analysis (EMA) was also performed, in which:
1) the structure was excited (random excitement) with an electrical signal having a frequency between 0 and 50 kHz;
2) the electrical input signal and the vibration responses in predefined points were measured using a 3D laser vibrometer;
3) the input and output signals were acquired and processed so as to obtain frequency response functions (FRF); and
4) a method of curve fitting in the time domain was used to extract the natural frequencies and the mode shapes of the tool during the vibration.

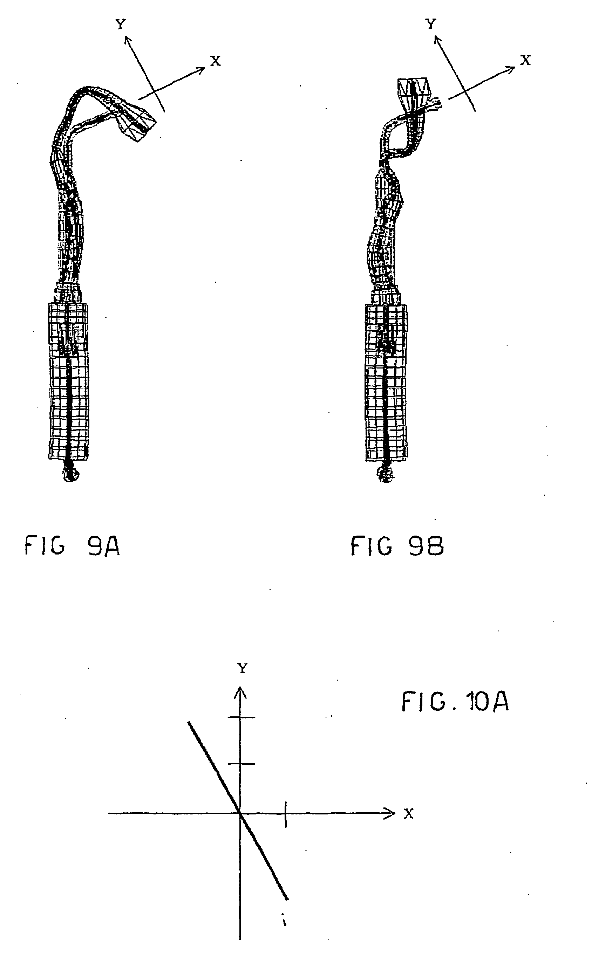

Initially the FE model of the structure of the tool 3 of the first embodiment (Figure 2-4) with a transducer was created and studied. Figures 9A and 9B show the nominal mode of vibration of said structure at a frequency between 25 - 30 kHz, during a compression cycle and an extension cycle, respectively. The modal shape of the longitudinal mode of the structure was amplified by a factor of 10,000 to see clearly the micrometric vibration of the tool.

The graph of Figure 10 illustrates the oscillating movement of the tool during a vibration cycle in a plane x-y, in which a ratio of 1:2 between the components x and y is detected. The tests performed using said tool showed that the calculated distribution of the movement offers an efficient penetration performance.

To help the surgeon during the preparation of the holes in the bone, the tool 103 of the second embodiment has been designed (Figures 6-9), having the flange or collar 137, in order to provide an indication of the level of penetration into the bone. The size and the positioning of the collar 137 have been chosen so as to have the least possible impact on the vibration performance of the tool. Therefore in this case also an FE model representing the structure of the tool 103 was realised.

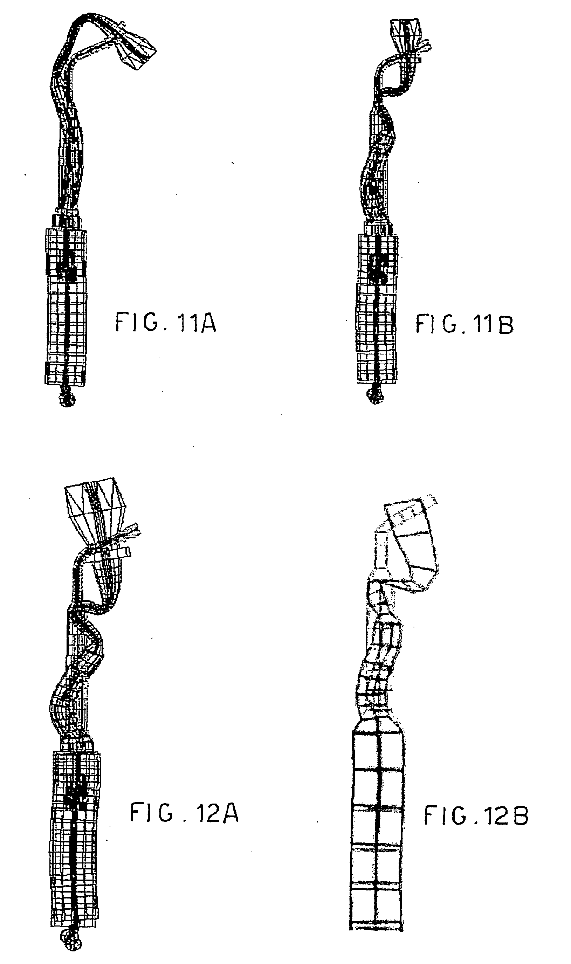

In Figures 11A and 11B the nominal vibration mode of the structure of the tool 103 with the transducer, at a frequency between 25 - 30 kHz, is illustrated during a compression cycle and an extension cycle, respectively, in which the modal shape of the longitudinal mode of the structure has been amplified by a factor of 10,000.

As is evident from the comparison of Figures 9A, 9B with Figures 11A, 11B, no significant variations in vibration characteristics have been noted between the tool without a collar 3 and the tool with a collar 103.

To provide validity of the simulations with FE models, an EMA was conducted with a 3D laser vibrometer (LDV). Figures 12A and 12B show respectively the data obtained from the FE analysis and from the EMA analysis with the 3D LDV vibrometer at a frequency between 25 - 30 kHz during an extension cycle. As is evident from the figures, there is an excellent correlation between the modal data obtained with FE analysis and those obtained with EMA analysis.

Numerous changes and modifications of detail within the reach of a person skilled in the art can be made to the present embodiments of the invention, without thereby departing from the scope of the invention, as set forth in the appended claims.

## Claims

1. A handpiece (1) for making holes in bone tissues, comprising a surgical tool (3; 103) provided with a tip or head (40; 140) adapted to penetrate the bone, said handpiece being an ultrasound handpiece adapted to make said tip of the tool (40; 140) vibrate at ultrasonic frequencies, said tip of the tool (40; 140) comprising a plurality of cutting elements (43) defining the profile of the hole to be made in the bone, and there being provided in the body of said tool a main channel (36) which ends in an outlet hole (44) opening into said tip (40; 140) for the passage of a cooling fluid, so as to cool the working area affected by the tool tip, wherein said cutting elements (43) of the tip of the tool (3; 103) are disposed radially with respect to said outlet hole (44), so as to give rise to radial channels (46) therebetween for the passage of the cooling fluid and in that, **characterized in that** on the outside surface of said tip (40; 140), there is provided a plurality of longitudinal channels (47) communicating with said radial channels (46) for the passage of the cooling fluid and the elimination of the removed material toward the outside.

2. A handpiece (1) according to claim 1, **characterised in that** it comprises a piezoelectric ultrasound transducer able to convert an electrical supply signal into a vibration at an ultrasonic frequency to set in vibration said tool (3; 103) connected thereto by means of a tang (30).

3. A handpiece (1) according to any one of the preceding claims, **characterised in that** said cutting elements (43) have at their periphery a cusp (45) facing towards the distal end of the tip (40; 140).

4. A handpiece (1) according to any one of the preceding claims, **characterised in that** said cutting elements (43) are substantially wedge-shaped with the cutting axis inclined with respect to the axis of the tip and converging towards the outlet hole (44), so as to give the tip (40; 140) a substantially concave cutting profile.

5. A handpiece (1) according to any one of the preceding claims, **characterised in that** said tool comprises a shank (32) which has a curved median portion (34) with an obtuse angle of between 90° and 180°, extremes excluded, preferably between 110° and 150°, which divides it into a first proximal part (32') and a second distal part (32") supporting the tip.

6. A handpiece (1) according to claim 5, **characterised in that** the axis of the proximal part (32') of the shank of the tool forms an angle α of about 20° with respect to the axis (X) of the tang (30) of the tool and the axis of the distal part (32") of the shank forms an angle β of about 13° with respect to the axis (X) of the tang.

7. A handpiece (1) according to claim 5, **characterised in that** the axis of the proximal part (32') of the shank of the tool coincides with the axis (X) of the tang (30) of the tool and the axis of the distal part (32") of the shank forms an angle θ of about 64° with respect to the axis (X) of the tang.

8. A handpiece (1) according to any one of the preceding claims, **characterised in that** the cutting elements (43) of the tip of the tool (3) are six in number, are arranged equidistant from each other by an angle of 60° and have peripheral cusps (45) which define a circumference having a diameter ranging from 1.8 to 2.5 mm, preferably 2.0 mm.

9. A handpiece (1) according to any one of claims 1 to 7, **characterised in that** the cutting elements (43) of the tip of the tool (103) are eight in number, are arranged equidistant from each other with an angle of 45° and have peripheral cusps (45) which define a circumference having a diameter ranging from 2.8 mm to 4.5mm, preferably 3.15 mm.

10. A handpiece (1) according to any one of the preceding claims, **characterised in that** said tool (103) further comprises a lateral outflow duct (138) communicating with the main duct (36) and opening near the tip (140) to send the cooling fluid into the side wall of the tip.

11. A handpiece (1) according to any one of the preceding claims, **characterised in that** said tool (103) further comprises a flange or ring or collar (137) situated on the shank (32), near the tip (140), having an outer profile substantially the same or slightly smaller than the outer profile of said cutting elements (43).

12. A handpiece (1) according to claims 10 and 11, **characterised in that** said lateral outlet duct (138) opens on the side surface of the tip, between said collar (137) and said cutting elements (43).

13. A handpiece (1) according to any one of claims 10 to 12, **characterised in that** the axis of said lateral outlet duct (138) of the tool (103) is inclined by an angle between 5° and 90°, preferably 45°, with respect to the axis of the main duct (36) of the tool.

14. An ultrasound handpiece (1) according to any one of claims 2 to 13, **characterised in that** said ultrasound transducer is supplied with an ultrasonic frequency carrier signal modulated with a low frequency modulating signal, consisting of burst.

## Patentansprüche

1. Handstück (1) zum Ausbilden von Löchern in Knochengeweben, umfassend ein chirurgisches Werkzeug (3; 103), das mit einer Spitze oder einem Kopf (40; 140) ausgestattet ist, die/der dazu geeignet ist, den Knochen zu durchdringen, wobei das Handstück ein Ultraschallhandstück ist, das dazu geeignet ist, die Spitze des Werkzeugs (40; 140) zum Vibrieren bei Ultraschallfrequenzen zu veranlassen, wobei die Spitze des Werkzeugs (40; 140) eine Vielzahl an Schneideelementen (43), die das Profil des im Knochen auszubildenden Lochs definiert, umfasst, wobei im Körper des Werkzeugs ein Hauptkanal (36), der in einer Auslassöffnung (44) endet, die sich in der Spitze (40; 140) öffnet, für den Durchtritt eines Kühlfluids bereitgestellt ist, um den von der Werkzeugspitze beeinflussten Arbeitsbereich zu kühlen, wobei die Schneideelemente (43) der Spitze des Werkzeugs (3; 103) radial in Bezug auf die Auslassöffnung (44) angeordnet sind, damit dazwischen radiale Kanäle (46) für den Durchtritt des Kühlfluids entstehen, **dadurch gekennzeichnet, dass** an der Außenoberfläche der Spitze (40; 140) eine Vielzahl an Längskanälen (47) bereitgestellt ist, die mit den radialen Kanälen (46) für den Durchtritt des Kühlfluids und die Beseitigung des entfernten Materials nach außen kommunizieren.

2. Handstück (1) nach Anspruch 1, **dadurch gekennzeichnet, dass** es einen piezoelektrischen Ultraschallwandler umfasst, der dazu imstande ist, ein elektrisches Versorgungssignal in eine Vibration mit einer Ultraschallfrequenz umzuwandeln, um das damit über eine Angel (30) verbundene Werkzeug (3; 103) in Vibration zu versetzen.

3. Handstück (1) nach einem der vorangegangenen Ansprüche, **dadurch gekennzeichnet, dass** die Schneideelemente (43) an ihrem Umfangsrand eine Zacke (45) aufweisen, die zum distalen Ende der Spitze (40; 140) weist.

4. Handstück (1) nach einem der vorangegangenen Ansprüche, **dadurch gekennzeichnet, dass** die Schneideelemente (43) im Wesentlichen keilförmig mit in Bezug auf die Achse der Spitze geneigter und zur Auslassöffnung (44) konvergierender Schneideachse sind, um der Spitze (40; 140) ein im Wesentlichen konkaves Schneideprofil zu verleihen.

5. Handstück (1) nach einem der vorangegangenen Ansprüche, **dadurch gekennzeichnet, dass** das Werkzeug einen Schaft (32) umfasst, der einen gebogenen Mittelabschnitt (34) mit einem stumpfen Winkel zwischen 90° und 180°, Grenzwerte nicht eingeschlossen, vorzugsweise zwischen 110° und 150°, aufweist, welcher ihn in einen ersten, proximalen Teil (32') und einen zweiten, distalen Teil (32"), der die Spitze trägt, unterteilt.

6. Handstück (1) nach Anspruch 5, **dadurch gekennzeichnet, dass** die Achse des proximalen Teils (32') des Schafts des Werkzeugs einen Winkel α von etwa 20° in Bezug auf die Achse (X) der Angel (30) des Werkzeugs bildet und die Achse des distalen Teils (32") des Schafts einen Winkel β von etwa 13° in Bezug auf die Achse (X) der Angel bildet.

7. Handstück (1) nach Anspruch 5, **dadurch gekennzeichnet, dass** die Achse des proximalen Teils (32') des Schafts des Werkzeugs mit der Achse (X) der Angel (30) des Werkzeugs deckungsgleich ist und die Achse des distalen Teils (32") des Schafts einen Winkel θ von etwa 64° in Bezug auf die Achse (X) der Angel bildet.

8. Handstück (1) nach einem der vorangegangenen Ansprüche, **dadurch gekennzeichnet, dass** die Spitze des Werkzeugs (3) sechs Schneidelemente (43) aufweist, die in gleichem Abstand in einem Winkel von 60° zueinander angeordnet sind und Umfangsrandzacken (45) aufweisen, die einen Umfang mit einem Durchmesser im Bereich von 1,8 bis 2,5 mm, vorzugsweise 2,0 mm, definieren.

9. Handstück (1) nach einem der Ansprüche 1 bis 7, **dadurch gekennzeichnet, dass** die Spitze des Werkzeugs (103) acht Schneidelemente (43) aufweist, die in gleichem Abstand in einem Winkel von 45° zueinander angeordnet sind und Umfangsrandzacken (45) aufweisen, die einen Umfang mit einem Durchmesser im Bereich von 2,8 bis 4,5 mm, vorzugsweise 3,15 mm, definieren.

10. Handstück (1) nach einem der vorangegangenen Ansprüche, **dadurch gekennzeichnet, dass** das Werkzeug (103) ferner einen seitlichen Ausströmungsgang (138) umfasst, der mit dem Hauptgang (36) kommuniziert und sich in der Nähe der Spitze (140) öffnet, um das Kühlfluid in die Seitenwand der Spitze zu führen.

11. Handstück (1) nach einem der vorangegangenen Ansprüche, **dadurch gekennzeichnet, dass** das Werkzeug (103) ferner einen Flansch oder Ring oder Kranz (137) umfasst, der am Schaft (32) in der Nähe der Spitze (140) angeordnet ist und ein Außenprofil aufweist, das im Wesentlichen das gleiche oder etwas kleiner als das Außenprofil der Schneideelemente (43) ist.

12. Handstück (1) nach den Ansprüchen 10 und 11, **dadurch gekennzeichnet, dass** sich der seitliche Ausströmungsgang (138) an der Seitenoberfläche der Spitze zwischen dem Kranz (137) und den Schneideelementen (43) öffnet.

13. Handstück (1) nach einem der Ansprüche 10 bis 12, **dadurch gekennzeichnet, dass** die Achse des seitlichen Ausströmungsgangs (138) des Werkzeugs (103) in Bezug auf die Achse des Hauptgangs (36) des Werkzeugs um einen Winkel zwischen 5° und 90°, vorzugsweise 45°, geneigt ist.

14. Ultraschallhandstück (1) nach einem der Ansprüche 2 bis 13, **dadurch gekennzeichnet, dass** der Ultraschallwandler mit einem Ultraschallfrequenzträgersignal, moduliert mit einem niederfrequenten Modulationssignal, bestehend aus Burst, versorgt wird.

## Revendications

1. Embout à main (1) pour réaliser des trous dans des tissus osseux, comprenant un outil chirurgical (3 ; 103) muni d'un embout ou d'une tête (40 ; 140) adapté pour pénétrer dans l'os, ledit embout à main étant un embout à main à ultrasons adapté pour faire vibrer ledit embout de l'outil (40; 140) à des fréquences ultrasoniques, ledit embout de l'outil (40; 140) comprenant une pluralité d'éléments de coupe (43) définissant le profil du trou à réaliser dans l'os, et un canal principal (36) étant prévu dans le corps dudit outil, qui débouche dans un trou de sortie (44) ouvrant dans ledit embout (40 ; 140) pour le passage d'un fluide de refroidissement, de façon à refroidir la zone de travail affectée par l'embout de l'outil, dans lequel lesdits éléments de coupe (43) de l'embout de l'outil (3 ; 103) sont disposés radialement par rapport audit trou de sortie (44), de façon à créer des canaux radiaux (46) entre ceux-ci pour le passage du fluide de refroidissement, et **caractérisé en ce que** sur la surface extérieure dudit embout (40; 140), il est prévu une pluralité de canaux longitudinaux (47) communiquant avec lesdits canaux radiaux (46) pour le passage du fluide de refroidissement et l'élimination du matériau retiré vers l'extérieur.

2. Embout à main (1) selon la revendication 1, **caractérisé en ce qu'**il comprend un transducteur à ultrasons piézoélectrique capable de convertir un signal d'alimentation électrique en une vibration à une fréquence ultrasonique pour faire vibrer ledit outil (3 ; 103) relié à celui-ci au moyen d'un tenon (30).

3. Embout à main (1) selon l'une quelconque des revendications précédentes, **caractérisé en ce que** lesdits éléments de coupe (43) présentent sur leur périphérie une cuspide (45) faisant face à l'extrémité distale de l'embout (40; 140).

4. Embout à main (1) selon l'une quelconque des revendications précédentes, **caractérisé en ce que** lesdits éléments de coupe (43) ont essentiellement une forme en coin avec l'axe de coupe incliné par rapport à l'axe de l'embout et convergeant en direction du trou de sortie (44), de façon à donner à l'embout (40 ; 140) un profil de coupe essentiellement concave.

5. Embout à main (1) selon l'une quelconque des revendications précédentes, **caractérisé en ce que** ledit outil comprend une tige (32) qui présente une partie médiane incurvée (34) avec un angle obtus compris entre 90° et 180°, extrêmes incluses, de préférence entre 110° et 150°, qui la divise en une première partie proximale (32') et une seconde partie distale (32") soutenant l'embout.

6. Embout à main (1) selon la revendication 5, **caractérisé en ce que** l'axe de la partie proximale (32') de la tige de l'outil forme un angle α d'environ 20° par rapport à l'axe (X) du tenon (30) de l'outil et l'axe de la partie distale (32") de la tige forme un angle β d'environ 13° par rapport à l'axe (X) de la tige.

7. Embout à main (1) selon la revendication 5, **caractérisé en ce que** l'axe de la partie proximale (32') de la tige de l'outil coïncide avec l'axe (X) du tenon (30) de l'outil et l'axe de la partie distale (32") de la tige forme un angle θ d'environ 64° par rapport à l'axe (X) de la tige.

8. Embout à main (1) selon l'une quelconque des revendications précédentes, **caractérisé en ce que** les éléments de coupe (43) de l'embout de l'outil (3) sont au nombre de six, sont disposés à équidistance l'un de l'autre d'un angle de 60° et présentent des cuspides périphériques (45) qui définissent une circonférence ayant un diamètre situé dans la plage de 1.8 à 2.5 mm, de préférence de 2.0 mm.

9. Embout à main (1) selon l'une quelconque des revendications 1 à 7, **caractérisé en ce que** les éléments de coupe (43) de l'embout de l'outil (103) sont au nombre de huit, sont disposés à équidistance l'un de l'autre d'un angle de 45° et présentent des cuspides périphériques (45) qui définissent une circonférence ayant un diamètre situé dans la plage de 2.8 à 4.5 mm, de préférence de 3.15 mm.

10. Embout à main (1) selon l'une quelconque des revendications précédentes, **caractérisé en ce que** ledit outil (103) comprend en outre un conduit d'écoulement de sortie latéral (138) communiquant avec le conduit principal (36) et ouvrant près de l'embout (140) pour envoyer le fluide de refroidissement dans la paroi latérale de l'embout.

11. Embout à main (1) selon l'une quelconque des revendications précédentes, **caractérisé en ce que** ledit outil (103) comprend en outre une bride ou une bague ou un collier (137) placé(e) sur la tige (32), près de l'embout (140), présentant un profil extérieur qui est essentiellement le même ou est légèrement plus petit que le profil extérieur desdits éléments de coupe (43).

12. Embout à main (1) selon les revendications 10 et 11, **caractérisé en ce que** ledit conduit d'écoulement de sortie latéral (138) ouvre sur la surface latérale de l'embout, entre ledit collier (137) et lesdits éléments de coupe (43).

13. Embout à main (1) selon l'une quelconque des revendications 10 à 12, **caractérisé en ce que** l'axe dudit conduit d'écoulement de sortie latéral (138) de l'outil (103) est incliné d'un angle compris entre 5° et 90°, de préférence 45°, par rapport à l'axe du conduit principal (36) de l'outil.

14. Embout à main (1) selon l'une quelconque des revendications 2 à 13, **caractérisé en ce que** ledit transducteur à ultra-sons reçoit un signal porteur de fréquences ultrasoniques modulé avec un signal de modulation basse fréquence, consistant en une salve.
